# EUROPEAN PATENT SPECIFICATION

(11) **EP 4 129 370 B1**
(45) Date of publication and mention of the grant of the patent: **17.06.2026**
(21) Application number: 21782238.6
(22) Date of filing: 30.03.2021
(51) Int. Cl.: A61M 5/34, A61M 5/158, A61M 5/31, A61M 5/32, A61M 5/315

(54) **NEEDLE BASE UNIT, NEEDLE BASE UNIT PRODUCTION METHOD, AND SYRINGE DEVICE**
NADELBASISEINHEIT, HERSTELLUNGSVERFAHREN FÜR NADELBASISEINHEIT UND SPRITZENVORRICHTUNG
UNITÉ DE BASE D'AIGUILLE, PROCÉDÉ DE PRODUCTION D'UNITÉ DE BASE D'AIGUILLE ET DISPOSITIF DE SERINGUE

(30) Priority: 31.03.2020 JP 2020063056
(43) Date of publication of application: 08.02.2023
(73) Proprietor: Asti Corporation, Hamamatsu-shi, Shizuoka 432-8056 (JP)
(72) Inventor: INOH, Akinori, Hamamatsu-shi, Shizuoka 432-8056 (JP); NONAKA, Isamu, Hamamatsu-shi, Shizuoka 432-8056 (JP); OGAI, Noriyuki, Hamamatsu-shi, Shizuoka 432-8056 (JP)
(74) Representative: Grünecker Patent- und Rechtsanwälte PartG mbB
(86) International application number: PCT/JP2021/013541
(87) International publication number: WO 2021/200946

(56) References cited:
- WO-A1-2011/040263
- WO-A1-2012/160852
- WO-A1-2014/162585
- JP-A- 2016 508 853
- JP-A- H0 852 213
- US-A- 3 035 616
- US-A- 5 902 277
- US-A- 5 964 737
- US-A1- 2016 279 345
- US-A1- 2017 197 035

## Description

### TECHNICAL FIELD

The present invention relates to a needle hub unit, a needle hub unit manufacturing method, and an injection device, and in particular, the present invention relates to those, by which the injection can be performed with the least possible waste of medicinal solution.

### BACKGROUND ART

A conventional injection needle is disclosed, for example, in Patent Document 1.

According to an invention of "medicinal solution injection device and an injection needle used for that device" described in Patent Document 1, there is a hub to which a needle is attached. A taper-shaped recess is formed in the hub, and the tip of a syringe is inserted into the taper-shaped recess. When the hub is attached to the syringe, first, a filling material is inserted into the taper-shaped recess, and thereafter, the tip of the syringe is inserted into the taper-shaped recess. A through hole communicating with the needle is formed in the filling material.

Further relevant prior art is described in US 3 035 616 A, WO 2012/160852 A1, US 2017/197035 A1, US 5 964 737 A, US 2016/279345 A1.

### REFERENCE DOCUMENTS OF CONVENTIONAL ARTPATENT DOCUMENT(S)

Patent Document 1: Official Gazette, Japan Patent Publication No. 2018-93906A.

### SUMMARY OF THE INVENTIONPROBLEMS TO BE SOLVED BY INVENTION

However, the configuration according to the conventional art as described above has the following problem.

That is, the filling material, which is to be inserted into the taper-shaped hub of the hub, includes the through hole formed therein, and consequently, there is a problem that, when the medicinal solution in the syringe is pushed out, a part of the medicinal solution remains unused in the through hole, and the medicinal solution cannot be used completely.

In the light of the above problem, it is an object of the present invention to provide a needle hub unit, a needle hub unit manufacturing method, and an injection device, by which the injection can be performed with the least possible waste of medicinal solution.

### MEANS TO SOLVE THE PROBLEM

The present invention relates to a needle hub unit defined in claim 1. Further it is provided an injection device comprising the needle hub unit. This device is defined in claim 7. Still further preferred embodiments are defined in the dependent claims.

In the following there are described some arrangements, however the scope of protection is defined by the claims. Thus some of the described arrangements may be helpful for understanding the present invention. According to arrangement 1, there is a needle hub unit, provided with: a needle hub; a recess provided in the needle hub and into which a tip portion of an injection cylinder is inserted; an elastic body internally provided in the recess and which fills a gap between the tip of the injection cylinder and the recess; and an injection needle inserted into the needle hub and penetrating through the elastic body.

Moreover, according to arrangement 2, with regard to the needle hub unit as specified in arrangement 1, the base end of the injection needle is formed in a shape of a needle, and a portion formed in the shape of the needle penetrates through and protrudes from the elastic body. Moreover, according to arrangement 3, with regard to the needle hub unit as specified in arrangement 1, the base end of the injection needle is aligned with a surface of the elastic body on the side of the injection cylinder.

Moreover, according to arrangement 4, with regard to the needle hub unit as specified in arrangement 3, the base end of the injection needle is cut to form a flat end.

Moreover, according to arrangement 5, with regard to the needle hub unit as specified in arrangement 3, the base end of the injection needle is formed in a shape of a needle, and a space is formed in the elastic body surrounding the base end of the injection needle.

Moreover, according to arrangement 6, with regard to the needle hub unit as specified in one arrangement among arrangement 1 to arrangement 5, an engagement portion is provided on the outer peripheral surface of the elastic body, and the engagement portion is engaged with an engagement portion provided on the side of the recess, so as to prevent the elastic body from dropping off.

Moreover, according to arrangement 7, with regard to the needle hub unit as specified in any one arrangement among arrangement 1 to arrangement5, the elastic body is provided with a thin thickness portion, and the injection needle penetrates through the thin thickness portion.

Moreover, according to arrangement 8, with regard to the needle hub unit as specified in any one arrangement among arrangement 1 to arrangement 5, the injection needles are provided in a plural number.

Moreover, according to arrangement 9, a needle hub unit manufacturing method, for manufacturing the needle hub unit as specified in any one arrangement among arrangement 1 to arrangement 5, is provided, characterized in that: the injection needle has been provided internally in the needle hub; and the elastic body is inserted into the recess of the needle hub, whereby a base portion of the injection needle penetrates through the elastic body.

Moreover, according to arrangement 10, with regard to the needle hub unit manufacturing method as specified in arrangement 9, recesses are provided in the central portion of both end surfaces, respectively disposed in the elastic body along the direction of the injection needle, whereby the base end of the injection needle penetrates through the positions of the respective recesses.

Moreover, according to arrangement 11, a needle hub unit manufacturing method, for manufacturing the needle hub unit as specified in any one arrangement among arrangement 1 to arrangement 5, is provided, characterized in that: the base portion of the injection needle and the elastic body have been formed integrally in advance, by penetration of the base portion of the injection needle through the elastic body; and the injection needle and the elastic body, formed integrally, is inserted into the recess of the needle hub.

And moreover, according to arrangement 12, there is an injection device provided with: the needle hub unit as specified in any one arrangement among arrangement 1 to arrangement 5; an injection cylinder inserted into the recess until the tip of the injection cylinder abuts on the elastic body; and a piston inserted into the injection cylinder until the tip of the piston abuts on the elastic body.

### EFFECT OF THE INVENTION

As described above, according to arrangement 1, there is a a needle hub unit, provided with: a needle hub; a recess provided in the needle hub and into which a tip portion of an injection cylinder is inserted; an elastic body internally provided in the recess and which fills a gap between the tip of the injection cylinder and the recess; and an injection needle inserted into the needle hub and penetrating through the elastic body. Therefore, during injection, the medicinal solution passes only through the inside of the injection needle, whereby the injection can be performed with the least possible waste of medicinal solution.

Moreover, according to arrangement 2, with regard to the needle hub unit as specified in arrangement 1, the base end of the injection needle is formed in a shape of a needle, and a portion formed in the shape of the needle penetrates through and protrudes from the elastic body. Therefore, during assembling of the needle hub unit, the needle can be penetrated through the elastic body easily, whereby the assembling thereof can be facilitated.

Moreover, according to arrangement 3, with regard to the needle hub unit as specified in arrangement 1, the base end of the injection needle is aligned with a surface of the elastic body on the side of the injection cylinder. Therefore, a piston of a syringe can be moved forward until reaching the surface of the elastic body on the side of the injection cylinder, whereby the injection can be performed with the further reduction of the waste of medicinal solution.

Moreover, according to arrangement 4, with regard to the needle hub unit as specified in arrangement 3, the base end of the injection needle is cut to form a flat end. Therefore, the effect of the present invention as described above can be secured further.

Moreover, according to arrangement 5, with regard to the needle hub unit as specified in arrangement 3, the base end of the injection needle is formed in a shape of a needle, and a space is formed in the elastic body surrounding the base end of the injection needle. Therefore, the effect of the present invention as described above can be secured further.

Moreover, according to arrangement 6, with regard to the needle hub unit as specified in any one arrangement among arrangement 1 to arrangement 5, an engagement portion is provided on the outer peripheral surface of the elastic body, and the engagement portion is engaged with an engagement portion provided on the side of the recess, so as to prevent the elastic body from dropping off. Therefore, the elastic body can be held securely.

Moreover, according to arrangement 7, with regard to the needle hub unit as specified in any one arrangement among arrangement 1 to arrangement 5, the elastic body is provided with a thin thickness portion, and the injection needle penetrates through the thin thickness portion. Therefore, the injection needle can be penetrated through the elastic body easily, whereby the assembling thereof can be facilitated.

Moreover, according to arrangement 8, with regard to the needle hub unit as specified in any one arrangement among arrangement 1 to arrangement 5, the injection needles are provided in a plural number. Therefore, the injection can be performed effectively.

Moreover, according to arrangement 9, a needle hub unit manufacturing method, for manufacturing the needle hub unit as specified in any one arrangement among arrangement 1 to arrangement 5, is provided, characterized in that: the injection needle has been provided internally in the needle hub; and the elastic body is inserted into the recess of the needle hub, whereby a base portion of the injection needle penetrates through the elastic body. Therefore, the assembling thereof can be facilitated.

Moreover, according to arrangement 0, with regard to the needle hub unit manufacturing method as specified in arrangement9, recesses are provided in the central portion of both end surfaces, respectively disposed in the elastic body along the direction of the injection needle, whereby the base end of the injection needle penetrates through the positions of the respective recesses. Therefore, the injection needle can be penetrated straight, through the center of the elastic body.

Moreover, according to arrangement 11, a needle hub unit manufacturing method, for manufacturing the needle hub unit as specififed in any one arrangement among arrangement 1 to arrangement 5, is provided, characterized in that: the base portion of the injection needle and the elastic body have been formed integrally in advance, by penetration of the base portion of the injection needle through the elastic body; and the injection needle and the elastic body, formed integrally, is inserted into the recess of the needle hub. Therefore, the assembling thereof can be facilitated.

And moreover, according to arrangement 12, there is an injection device provided with: the needle hub unit as specified in any one arrangement among arrangement1 to arrangement 5; an injection cylinder inserted into the recess until the tip of the injection cylinder abuts on the elastic body; and a piston inserted into the injection cylinder until the tip of the piston abuts on the elastic body. Therefore, the waste of medicinal solution can be eliminated.

### BRIEF DESCRIPTION OF DRAWINGS

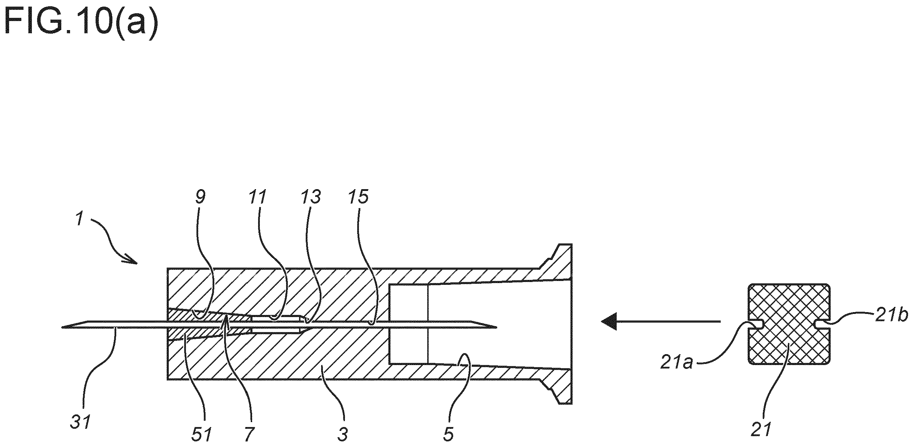

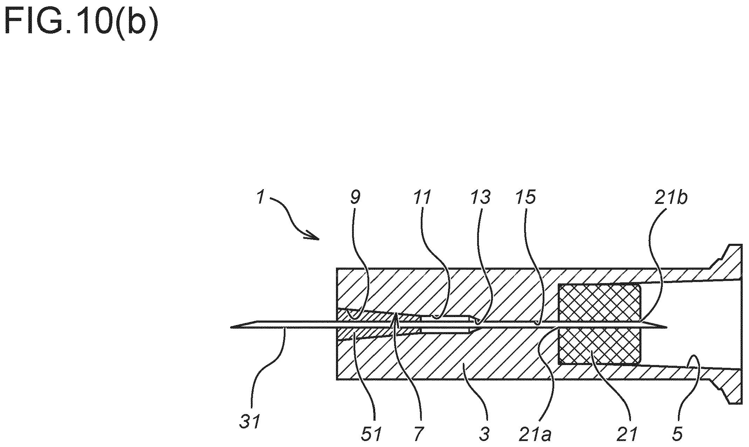

From the following arrangements the arrangement shown in Figure 10 is an embodiment of the present invention. The further arrangements disclosed in the figures are helpful for understanding the present invention.
[Figure 1] Fig. 1 is a longitudinal sectional view according to a first arrangement, showing a needle hub unit into which a tip of an injection cylinder is inserted.
[Figure 2] Fig. 2 are views according to the first arrangement, showing procedure for of assembling of the needle hub unit, in which: Fig. 2 (a) is a longitudinal sectional view showing a state that an elastic body is about to be inserted into the needle hub; and Fig. 2 (b) is a longitudinal sectional view showing a state that the elastic body has been inserted into the needle hub.
[Figure 3] Fig. 3 is a longitudinal sectional view according to a second arrangement, showing a needle hub unit into which a tip of an injection cylinder is inserted.
[Figure 4] Fig. 4 are views according to the second arrangement, showing procedure for assembling of the needle hub unit, in which: Fig. 4 (a) is a longitudinal sectional view showing a state that an elastic body is about to be inserted into the needle hub; and Fig. 4 (b) is a longitudinal sectional view showing a state that the elastic body has been inserted into the needle hub.
[Figure 5] Fig. 5 is a longitudinal sectional view of a needle hub unit according to a third arrangement.
[Figure 6] Fig. 6 is a longitudinal sectional view according to a fourth arrangement, showing a needle hub unit into which a tip of an injection cylinder is inserted.
[Figure 7] Fig. 7 is a longitudinal sectional view of a needle hub unit according to a fifth arrangement.

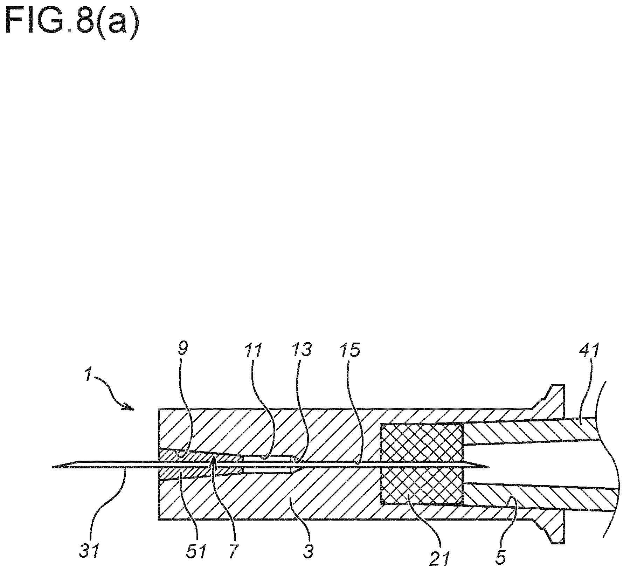

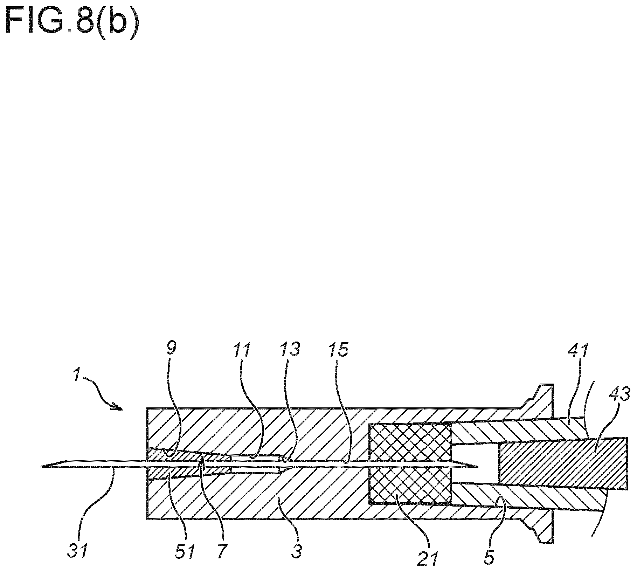

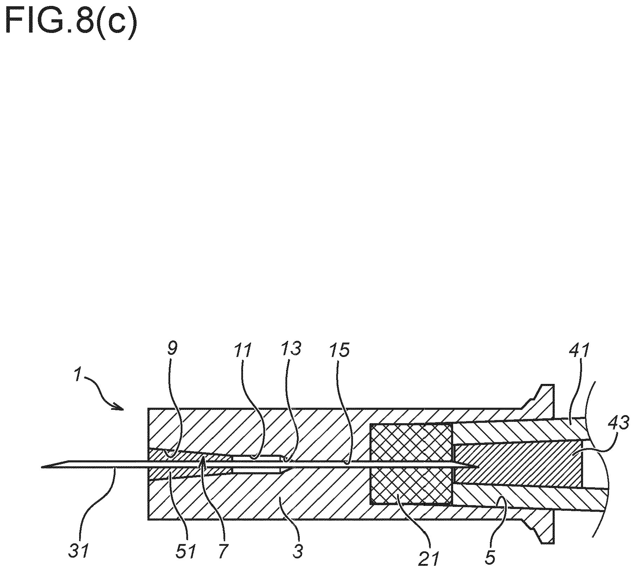

[Figure 8] Fig. 8 are views according to a sixth arrangement, in which: Fig. 8 (a) is a sectional view showing a state that, an elastic body is inserted into a needle hub, and thereafter an injection cylinder is inserted therein; Fig. 8 (b) is a sectional view showing a state that. the elastic body is inserted into the needle hub, and thereafter the injection cylinder is inserted therein, and thereafter, a piston is inserted therein; and Fig. 8 (c) is a sectional view showing a state that, the elastic body is inserted into the needle hub, and thereafter the injection cylinder is inserted therein, and thereafter, the piston is inserted therein until the piston abuts on the elastic body.
[Figure 9] Fig. 9 is a sectional view according to a seventh arrangement, showing a state that, an elastic body is inserted into a needle hub, and thereafter an injection cylinder is inserted therein, and thereafter, a piston is inserted therein until the piston abuts on the elastic body.
[Figure 10] Fig. 10 are views according to an eighth arrangement, in which: Fig. 10 (a) is a longitudinal sectional view showing a state that an elastic body is about to be inserted into a needle hub; and Fig. 10 (b) is a longitudinal sectional view showing a state that the elastic body has been inserted into the needle hub.

### MODE(S) FOR CARRYING OUT THE INVENTION

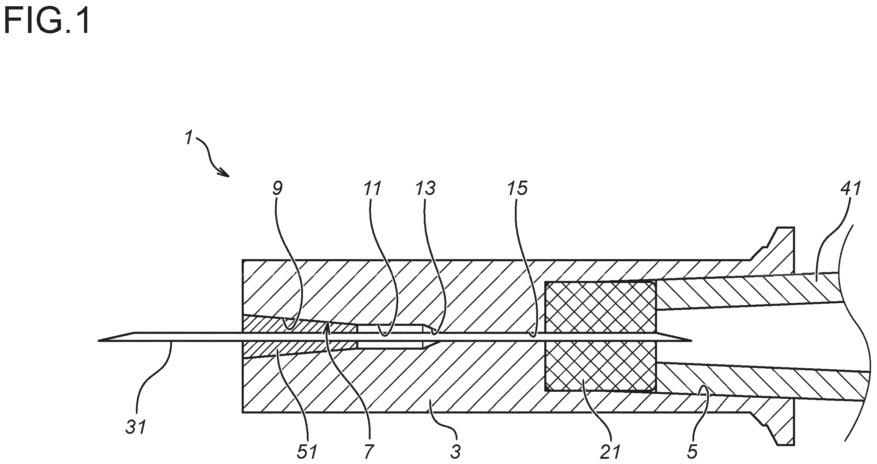

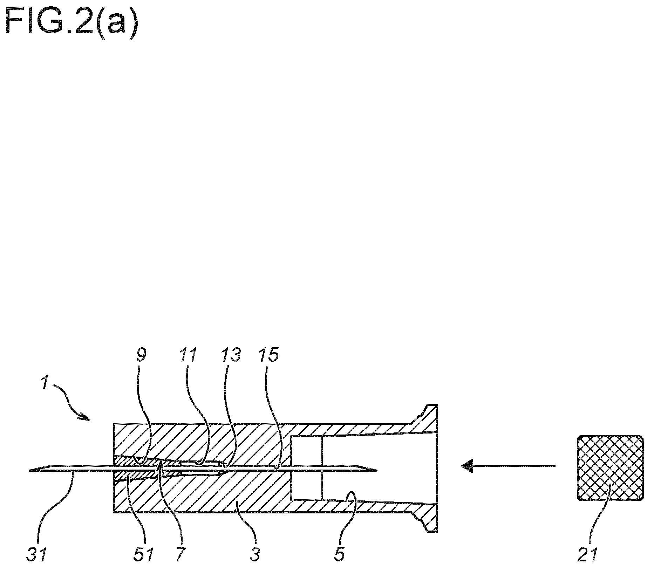

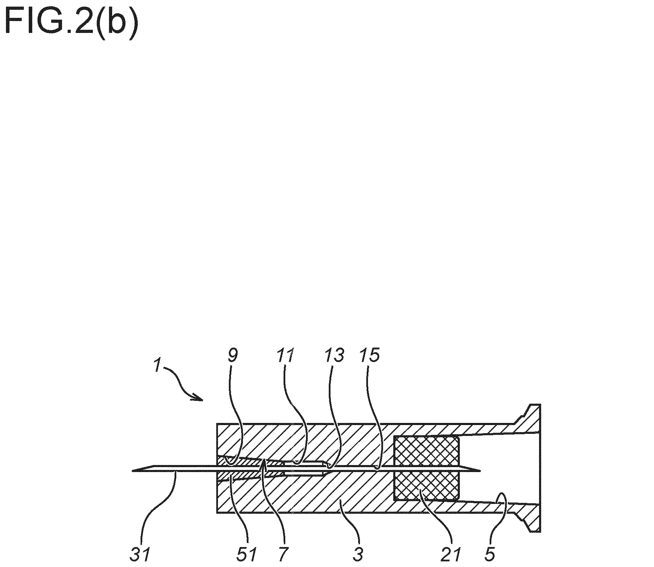

Now, a first arrangement will be explained with reference to Fig. 1 and Fig. 2.

First, as illustrated in Fig. 1, a needle hub unit 1 includes a needle hub 3. An injection cylinder insertion recess 5 is formed on the right side of the needle hub 3 as shown in Fig. 1. The injection cylinder insertion recess 5 is in a taper shape, of which diameter increases gradually toward the side of an opening (on the right side of Fig. 1).

Moreover, a needle insertion through hole 7 is formed on the left side of the needle hub 3. The needle insertion through hole 7 and the injection cylinder insertion recess 5 are communicating with each other. The left side of the needle insertion through hole 7 as shown in Fig. 1 constitutes a taper-shaped portion 9, of which diameter increases gradually toward the side of an opening (on the left side of Fig. 1). A first straight portion 11 is formed, of which diameter remains unchanged at the same diameter as that of the tape-shaped portion 9 on the side opposite to the opening (at the right end of Fig. 1), in the vicinity of the center of the needle insertion through hole 7 in the right and left directions of Fig. 1 . The right side of the first straight portion 11 of Fig. 1 constitutes a diameter decreasing portion 13, of which diameter decreases gradually toward the right side of Fig. 1. A second straight portion 15 as an open hole, of which diameter remains unchanged at the same diameter as that of the diameter decreasing portion 13 on the side opposite to the opening (at the right end of Fig. 1), is formed in the needle insertion through hole 7, on the right side of the diameter decreasing portion 13 as shown in Fig 1.

Moreover, the needle unit 1 includes an elastic body 21. The elastic body 21 is made of, for example, silicone rubber, butyl rubber or thermoplastic elastomer, and is inserted until reaching the deepest end (at the left end of Fig. 1) of the injection cylinder insertion recess 5 of the needle hub 3.

Moreover, the needle hub unit 1 includes an injection needle 31. The injection needle 31 is inserted into the needle insertion through hole 7 of the needle hub 3, and is installed in the needle hub 3. Moreover, the base end side (on the right side of Fig. 1) of the injection needle 31 penetrates through the elastic body 21, and protrudes into the inside of the injection cylinder insertion recess 5. Moreover, the both ends of the injection needle 31 are formed in the shape of needles, respectively.

The needle hub unit 1 is attached to the injection cylinder 41, with the press fitting of the tip of the injection cylinder 41 into the injection cylinder insertion recess 5. As illustrated in Fig. 1, the injection cylinder 41 is press-fitted into the injection cylinder insertion recess 5, until the top of the injection cylinder 41 depresses the elastic body 21. Moreover, when the needle hub unit 1 is attached to the injection cylinder 41, the base end side of the injection needle 31 (on the right side of Fig. 1) protrudes into the inside of the injection cylinder 41.

An adhesive 51 is filled into a gap between the injection needle 31 and the inner peripheral surface of the needle insertion through hole 7, and by means of the adhesive 51, the injection needle 31 is held in the injection cylinder 41.

Next, the function of the first arrangement will be explained.

The assembling of the needle hub unit 1 is performed as shown in Fig. 2.

First, as illustrated in Fig. 2 (a) , the injection needle 31 is inserted into the needle insertion through hole 7 of the needle hub 3, and the adhesive 51 is filled in the gap between the injection needle 31 and the inner peripheral surface of the needle insertion through hole 7.

Next, as illustrated in Fig. 2 (b), the elastic body 21 is inserted into the injection cylinder insertion press-fitting recess 5 of the needle hub 3. In such a situation, the injection needle 31 penetrates through the elastic body 21, and the base end side of the injection needle 31 (on the right side of Fig. 2 (b) ) protrudes into the inside of the injection cylinder insertion recess 5.

When the needle hub unit 1 is used, as illustrated in Fig. 1 , the injection cylinder 41 is press-fitted into the injection cylinder insertion recess 5, until the tip of the injection cylinder 41 depresses the elastic body 21.

Next, the effect of the first arrangement will be explained.

First, there is the elastic body 21 in the injection cylinder insertion recess 5 of the needle hub 3, the injection needle 31 penetrates through the elastic body 21, and when the needle hub unit 1 is attached to the injection cylinder 41, the base end side of the injection needle 31 (on the right side of Fig. 1) protrudes into the inside of the injection cylinder 41. Consequently, a medicinal solution in the injection cylinder 41 is injected by passing only through the inside of the injection needle 31, and no medicinal solution is left unused in the needle hub unit 1, and therefore, the injection can be performed with the least possible waste of the medicinal solution.

Moreover, since the protruding portion on the base end side of the injection needle 31 is formed in the shape of needle, there is no medicinal solution left in that portion.

And moreover, since the both ends of the injection needle 31 are in the form of needles, respectively, when assembling of the needle hub unit 1, the injection needle 31 can penetrate through the elastic body 21 easily, whereby the assembling thereof can be facilitated.

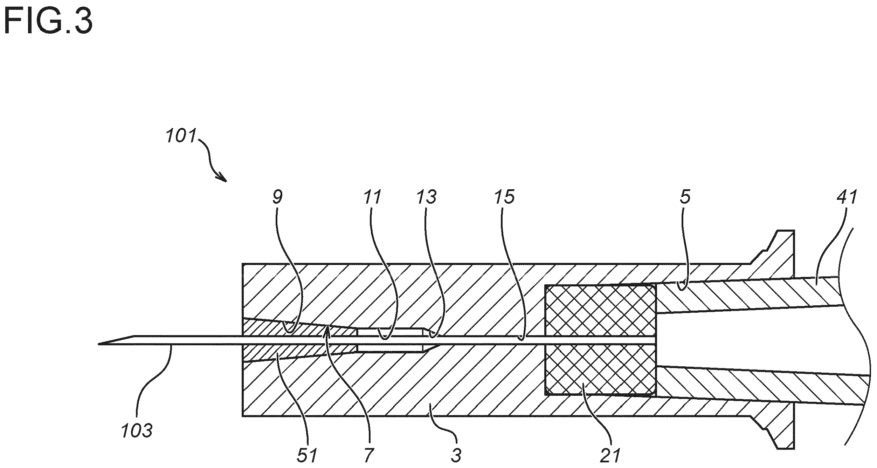

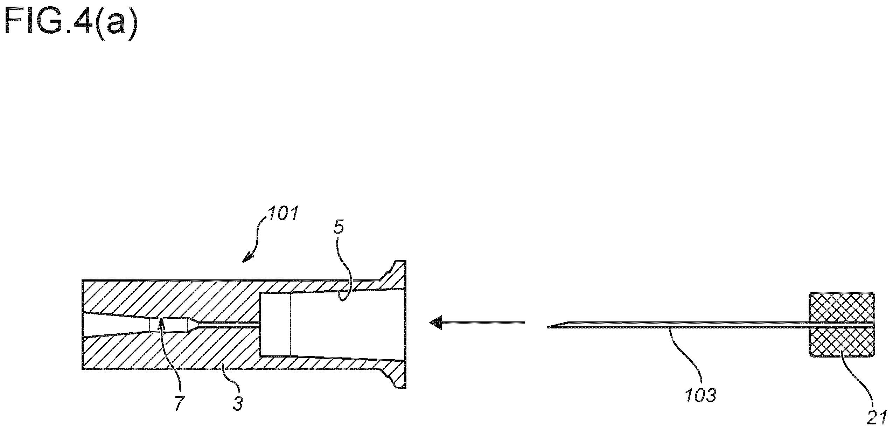

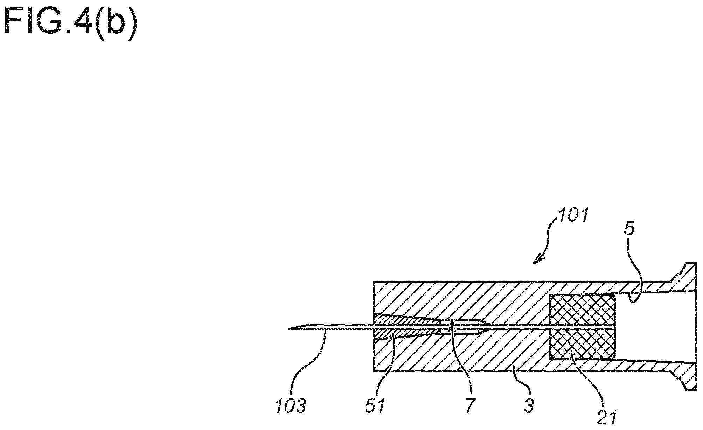

Next, a second arrangement will be explained with reference to Fig. 3 and Fig. 4.

The structure of a needle hub unit 101 according to the second arrangement is substantially the same as that of the needle hub unit 1 of the first arrangement. However, there is a different feature that, an injection needle 103 having a different structure is installed in the needle hub 3. With regard to the injection needle 103, only the tip side (on the left side of Fig. 3) is formed in the shape of needle, and the base end side (on the right side of Fig. 3 ) is cut to form a flat end. Further, with regard to the injection needle 103, the base end side (on the right side of Fig. 3 ) is aligned with the end surface of the elastic body 21 on the side of the injection cylinder 41 (on the right side of Fig. 3 ), and does not protrude into the side of the injection cylinder 41.

Next, the function of the second arrangement will be explained.

The assembling of the needle hub unit 101 is performed as shown in Fig. 4.

First, as illustrated in Fig. 4 (a), the tip side of the injection needle 31 (on the left side of Fig. 4 (a)) is pierced from the right side of the elastic body 21 as shown in Fig. 4 (a), and is moved until the end surface of the injection needle 31 on the base end surface (on the right side of Fig. 4 (a)) becomes aligned with the end surface of the elastic body 21 on the right side of Fig. 4 (a), to form a uniform surface with each other.

Next, as illustrated in Fig. 4 (b), the tip side of the injection needle 31 (on the left side of Fig. 4 (b)) is inserted into the needle insertion through hole 7, from the side of the injection cylinder insertion recess 5 of the needle hub 3 (on the right side of Fig. 4 (b)), and the elastic body 21 is inserted into the deepest end of the injection cylinder insertion recess 5 (on the left side of Fig. 4 (b)). Thereafter, the adhesive 51 is filled in the gap between the injection needle 31 and the inner peripheral surface of the needle insertion through hole 7.

Furthermore, in the case of the second arrangement, in addition to the same function and effect as those of the first arrangement, the following function and effect can be exhibited. That is, the base end side of the injection needle 103 (on the right side of Fig. 3 ) is aligned with the end surface of the elastic body 21 on the side of the injection cylinder 41 (on the right side of Fig. 3 ), and the injection needle does not protrude into the side of the injection cylinder 41. Consequently, a piston (not shown) of the injection cylinder 41 can move forward until reaching the end surface of the elastic body 21 on the side of the injection cylinder 41 (on the right side of Fig. 3 ), whereby the medicinal solution can be injected with the further reduction of the waste of medicinal solution. The piston is normally composed of flexible materials, and therefore, even in the case of the structure according to the first arrangement, the piston may move forward until reaching the end surface of the elastic body 21 on the side of the injection cylinder 41 (on the right side of Fig. 3 ). However, where the piston is composed of rigid materials, the injection needle 41 protruding into the inside of the injection cylinder 41 is unable to pierce into the piston, and the further depressing of the piston is prohibited. The present arrangement is effective in such a situation.

It should be noted that, in the drawings, the same reference signs will be allotted to the elements common to the first arrangement and the second arrangement, and the explain thereof will be omitted.

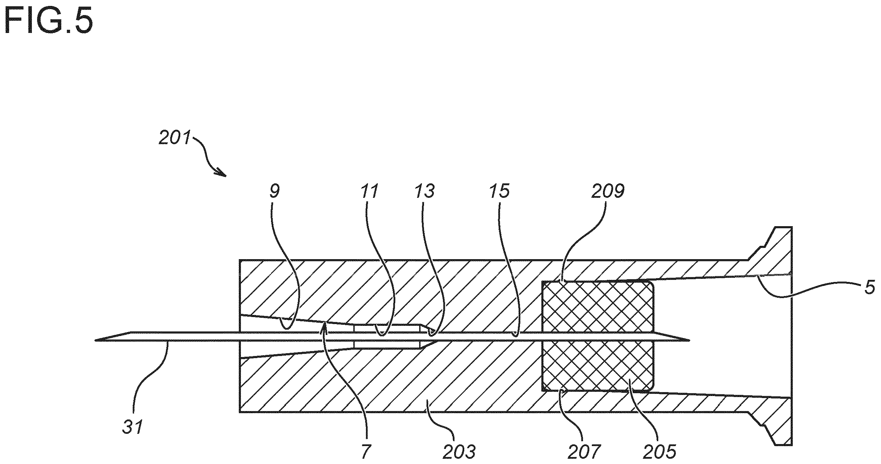

Next, a third arrangement will be explained with reference to Fig. 5.

The structure of a needle hub unit 201 according to the third arrangement is substantially the same as that of the needle hub unit 1 of the first arrangement. However, there is a different feature that, a needle hub 203 and an elastic body 205, respectively having different structures, are used. The structure of the needle hub 203 is substantially the same as that of the needle hub 3 of the first arrangement, but an elastic engagement recess 207 in an annular shape is formed on the inner peripheral surface of the bottom side of the injection cylinder insertion recess 5 (on the left side of Fig. 5), and an elastic body engagement projection 209 is formed on the side circumferential surface of the elastic body 205. Furthermore, when the elastic body 205 is installed in the injection cylinder insertion recess 5, the elastic body engagement recess 207 and the elastic body engagement projection 209 are engaged with each other.

Next, according to the third arrangement, in addition to the effect and function of the first arrangement, the following function and effect can be further exhibited. That is, when the elastic body 205 is installed in the injection cylinder insertion recess 5, the elastic body engagement recess 207 and the elastic body engagement projection 209 are engaged with each other, and consequently, the elastic body 205 can be held securely.

It should be noted that, in the drawings, the same reference signs will be allotted to the elements common to the first arrangement and the third arrangement, and the explain thereof will be omitted.

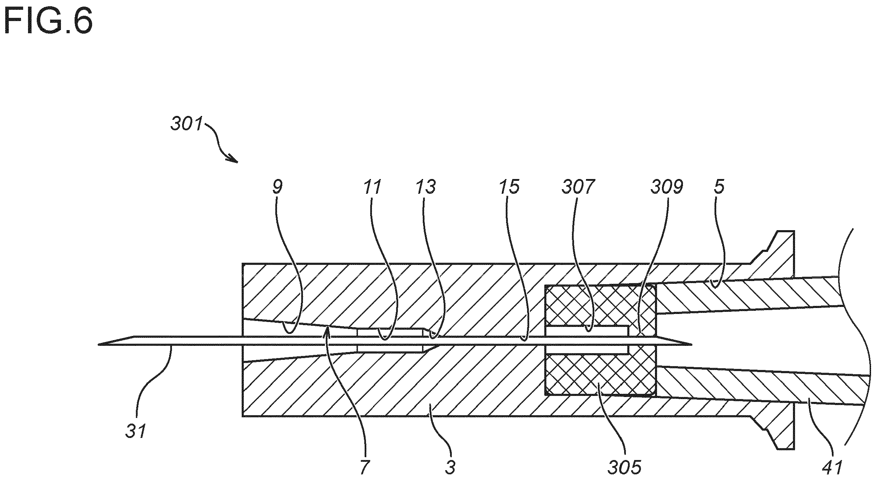

Next, a fourth arrangement will be explained with reference to Fig. 6.

The structure of a needle hub unit 301 according to the fourth arrangement is substantially the same as that of the needle hub unit 1 of the first arrangement. However, there is a different feature that, an elastic body 305 having a different structure is used. With regard to the elastic body 305, a recess 307, having an opening toward the bottom side of the injection cylinder insertion recess 5 (on the left side of Fig. 6), is formed, and a thin thickness portion 309 is formed on the bottom side of the recess 307 (on the right side of Fig. 6 ). The base end side of the injection needle 31 (on the right side of Fig. 6) penetrates through the thin thickness portion 309, and is exposed on the side of the injection cylinder insertion recess 5.

Next, according to the fourth arrangement, in addition to the effect and function of the first arrangement, the following function and effect can be further exhibited. That is, the base end side of the injection needle 31 (on the right side of Fig. 6 ) penetrates through the thin thickness portion 309 of the elastic body 305, and is exposed on the side of the injection cylinder insertion recess 5, and consequently, the injection needle 31 can penetrate through the elastic body 305 easily. Therefore, when the needle hub unit 301 is assembled, the installation of the elastic body 305 in the injection cylinder insertion recess 5 can be performed easily, and consequently, the assembling of the needle hub unit 301 can be facilitated.

It should be noted that, in the drawings, the same reference signs will be allotted to the elements common to the first arrangement and the fourth arrangement, and the explain thereof will be omitted.

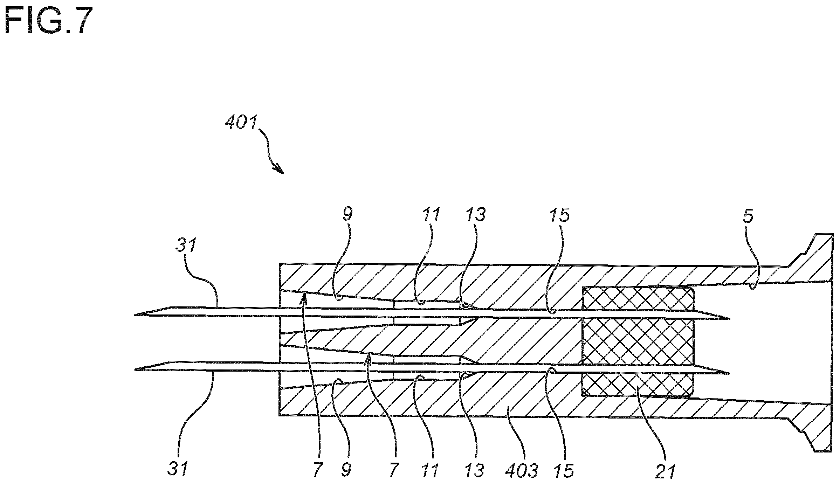

Next, a fifth arrangement will be explained with reference to Fig. 7.

The structure of a needle hub unit 401 according to the fifth arrangement is substantially the same as that of the needle hub unit 1 of the first arrangement. However, there is a different feature that, a needle hub 403 having a different structure is used. The structure of the needle hub 403 is substantially the same as that of the needle hub 3 of the first arrangement, but a plurality of (in the fifth arrangement, two) needle insertion through holes 7 are formed, and a plurality of (in the fifth arrangement, two) injection needles 31 are installed.

Next, according to the fifth arrangement, in addition to the effect and function of to the first arrangement, the following function and effect can be further exhibited. That is, since a plurality of (in the fifth arrangement, two) injection needles 31 are installed, the injection can be performed more effectively.

It should be noted that, in the drawings, the same reference signs will be allotted to the elements common to the first arrangement and the fifth arrangement, and the explain thereof will be omitted.

Next, a sixth arrangement will be explained with reference to Fig. 8. In the case of the first arrangement, there is no specific requirement for the structure of the piston, which is inserted into the injection cylinder. However, in the present arrangement, as illustrated in Fig. 8 (c), there is a prerequisite that a piston 43, which is inserted into the injection cylinder 41, can be inserted until the tip of the piston 43 abuts on the elastic body 21.

For reference, the other structure is substantially the same as that of the first arrangement.

According to the above structure, when the injection needle 31 is put into a medicinal solution bottle (not shown) and the piston 43 is pulled, the medicinal solution in the medicinal solution bottle is sucked into the injection cylinder 41.

For reference, if the injection needle 31 is thin, the medicinal solution is sucked into the injection cylinder 41 by using another needle hub 3 provided with a thick injection needle 31, and thereafter, the replacement to the needle hub 3 provided with the thick injection needle 31 is performed.

Next, the injection needle 31 is punctured into a predetermined position of a human (not shown), and as illustrated in Fig. 8 (b), the piston 43 is depressed. Thereafter, as illustrated in Fig. 1 (c), the piston 43 is depressed until the tip surface of the piston 43 abuts on the surface of the elastic body 21. As a result, all the medicinal solution in the injection cylinder 41 can be ejected completely.

Therefore, substantially the same effect as that of the first arrangement can be accomplished, and in addition, since the piston 43 can be depressed until abutting on the elastic body 21, the waste of medicinal solution can be reduced further.

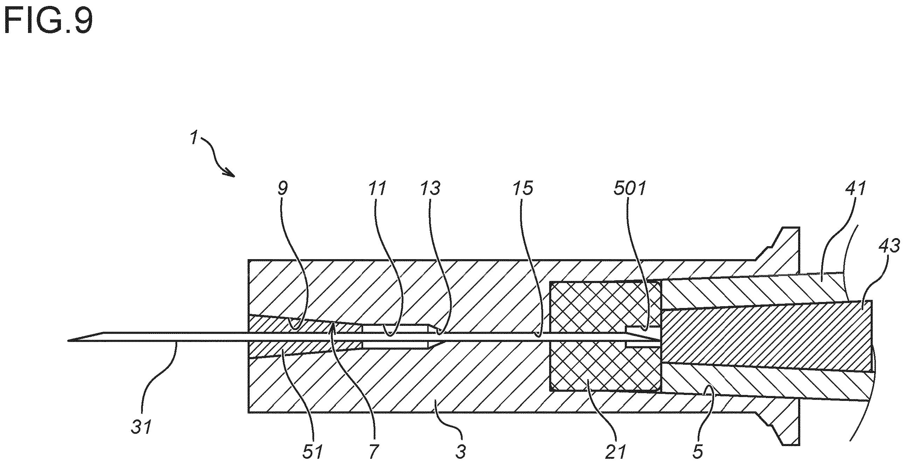

Next, a seventh arrangement will be explained with reference to Fig. 9. In the present arrangement, substantially the same as the case of the second arrangement, the base end side of the injection needle 103 (on the right side of Fig. 9) is aligned with the end surface of the elastic body 21 on the side of the injection cylinder 41 (on the right side of Fig. 9), and does not protrude into the side of the injection cylinder 41. However, and moreover, the base end of the injection needle 31 is not in a flat shape, but has been cut off obliquely. Moreover, a recess 501 is formed on the end surface of the elastic body 21, surrounding the base end portion of the injection needle 31.

The other structure is substantially the same as that of the second arrangement as described above, thus the same reference signs will be allotted to the common elements in the drawing, and the explain thereof will be omitted.

Since the base end side of the injection needle 103 (on the right side of Fig. 9 ) is aligned with the end surface of the elastic body 21 on the side of the injection cylinder 41 (on the right side of Fig. 9 ), the piston 43 can be depressed until abutting on the elastic body 21. Moreover, the base end side of the injection needle 31 is not in a flat shape, but has been cut off obliquely, and consequently, for the purpose of manufacturing, the same method as that of the first arrangement can be adopted. Moreover, since the recess 501 is formed, the medicinal solution can be introduced into the injection needle 31 securely.

Next, an eighth arrangement, which is an embodiment of the present invention will be explained with reference to Fig. 10. With regard to the structure of the first arrangement as described above, the present arrangement has a structure that recesses 21a, 21b are provided at the center position of the both end surfaces along the insertion direction of the elastic body 21. For reference, the other structure is substantially the same as that of the first arrangement as described above, thus the same reference signs will be allotted to the common elements in the drawing, and the explain thereof will be omitted.

According to the structure as described above, since the recesses 21a, 21b are provided at the center position of the both end surfaces along the insertion direction of the elastic body 21, there is a merit that the base end portion of the injection needle 31 is guided to the center of the elastic body 21, and straight penetration thereof at the correct angle can be performed. In addition, substantially the same effect as that of the first arrangement can be accomplished.

For reference, each of the recesses 21a, 21b is substantially in the same size as, or slightly larger than, the outer diameter of the injection needle 31. Therefore, when the elastic body 21 is deformed by the injection needle, the recesses 21a, 21b becomes filled up.

The present invention is not limited to the first to the eighth arrangements as described above, but is limited by the appended independent claims. Optional features are part of the appended dependent claims.

Any other rubber, elastomer, etc., may be used as an elastic member.

The needle hub and the injection needle may be produced integrally by means of insert molding. Various materials, sizes, and numbers may be adopted for each of the structural elements.

Furthermore, the structures shown in the drawings are merely for example purposes only.

### INDUSTRIAL APPLICABILITY

The present invention relates to the needle hub unit, the needle hub unit manufacturing method, and the injection device, and in particular, the present invention relates to those, by which the injection can be performed with the least possible waste of medicinal solution. The present invention is suitable, for example, for a syringe for the injection of Botox (trademark, botulinum toxin type A).

### EXPLANATION OF REFERENCE NUMERALS AND SIGNS

1: Needle Hub Unit
5: Injection Cylinder Insertion Recess
21: Elastic Body
41: Injection Cylinder
31: Injection Needle
101: Needle Hub Uni
103: Injection Needle
201: Needle Hub Unit
207: Elastic Body Engagement Recess
209: Elastic Body Engagement Projection
301: Needle hub Unit
305: Elastic Body
309: Thin Thickness Portion
401: Needle Hub Unit

## Claims

1. A needle hub unit (1) provided with:
a needle hub (3);
a recess (5) provided in a base end side of the needle hub (3) and into which a tip portion of an injection cylinder (41) is inserted;
a needle insertion through hole (7) provided on a tip side of the needle hub (3) and communicating with the recess (5);
an injection needle (31) inserted into the recess (5) and the needle insertion through hole (7); and
an elastic body (21), which is internally provided in the recess (5) from a side of an opening of the recess (5) and a tip surface of the elastic body (21) abuts on a bottom surface of the recess (5), and which fills a gap between the tip of the injection cylinder (41) and the recess (5),
wherein,
a tip and a base end of the injection needle (31) is formed in a shape of a needle;
guide recesses (21a, 21b) for guiding the injection needle (31) are provided in a center position, respectively, on each end surface along a direction toward the injection needle (31) of the elastic body (21);
an adhesive (51) is filled inside the needle insertion through hole (7); and
the base end of the injection needle (31) is punctured into the elastic body (21) via the guide recess (21a), and penetrates through the elastic body (21) via the guide recess (21b).

2. The needle hub unit (1) as claimed in Claim 1, wherein,
the guide recesses (21a, 21b) are provided, respectively, at a diameter substantially in a same size as, or slightly larger than, an outer diameter of the injection needle (31).

3. The needle hub unit (1) as claimed in Claim 1, wherein,
the guide recesses (21a, 21b) are filled up when the elastic body (21) is deformed by the injection needle (31).

4. The needle hub unit (1) as claimed in Claim 1 wherein
the needle hub (3) is configured by a single member.

5. The needle hub unit (1) as claimed in any one Claim among Claim 1 to Claim 4, wherein,
an engagement portion (209) is provided on the outer peripheral surface of the elastic body (21), and the engagement portion is engaged with an engagement portion (207) provided on the side of the recess, so as to prevent the elastic body (21) from dropping off.

6. A needle hub unit manufacturing method, for manufacturing the needle hub unit (1) as claimed in any one Claim among Claim 1 to Claim 5, wherein,
the injection needle (31) has been provided internally in the recess (5) and the needle insertion through hole (7) of the needle hub (3);
an adhesive (51) is filled inside the needle insertion through hole (7); and
the elastic body (21) is inserted into the recess (5) of the needle hub (3), whereby a base portion of the injection needle (31) penetrates through the elastic body (21).

7. An injection device provided with:
the needle hub unit (1) as claimed in any one Claim among Claim 1 to Claim 5;
an injection cylinder (41) inserted into the recess (5) until the tip of the injection cylinder (41) abuts on the elastic body (21); and
a piston (43) inserted into the injection cylinder (41) until the tip of the piston abuts on the elastic body (21).

## Patentansprüche

1. Eine Nadelhülseneinheit (1), die Folgendes umfasst:
eine Nadelhülse (3);
eine Aussparung (5), die an der Basisendseite der Nadelhülse (3) vorgesehen ist und in die ein Spitzenabschnitt eines Injektionszylinders (41) eingeführt ist;
ein Nadeleinführungsdurchgangsloch (7), das an der Spitzenseite der Nadelhülse (3) vorgesehen ist und mit der Aussparung (5) in Verbindung steht;
einer Injektionsnadel (31), die in die Aussparung (5) und die Nadeleinführungsdurchgangsöffnung (7) eingeführt ist; und
einem elastischen Körper (21), der von einer Seite einer Öffnung der Aussparung (5) her in der Aussparung (5) angeordnet ist und dessen Spitzenfläche an einer Bodenfläche der Aussparung (5) anliegt, und der einen Spalt zwischen der Spitze des Injektionszylinders (41) und der Aussparung (5) ausfüllt,
wobei
eine Spitze und ein Basisende der Injektionsnadel (31) in Form einer Nadel ausgebildet sind;
Führungsausnehmungen (21a, 21b) zum Führen der Injektionsnadel (31) sind jeweils an einer mittleren Position an jeder Endfläche entlang einer Richtung zur Injektionsnadel (31) hin am elastischen Körper (21) vorgesehen;
ein Klebstoff (51) ist in das Nadel-Einführloch (7) eingefüllt; und
das Basisende der Injektionsnadel (31) wird über die Führungsausnehmung (21a) in den elastischen Körper (21) eingestochen und durchdringt den elastischen Körper (21) über die Führungsausnehmung (21b).

2. Die Nadelhülseneinheit (1) nach Anspruch 1, wobei
die Führungsausnehmungen (21a, 21b) jeweils mit einem Durchmesser vorgesehen sind, der im Wesentlichen der Größe des Außendurchmessers der Injektionsnadel (31) entspricht oder geringfügig größer ist als dieser.

3. Die Nadelhülseneinheit (1) nach Anspruch 1, wobei
die Führungsausnehmungen (21a, 21b) aufgefüllt werden, wenn der elastische Körper (21) durch die Injektionsnadel (31) verformt wird.

4. Die Nadelhülseneinheit (1) nach Anspruch 1, wobei
die Nadelhülse (3) aus einem einzigen Bauteil ausgebildet ist.

5. Die Nadelhülseneinheit (1) nach einem der Ansprüche 1 bis 4, wobei
ein Eingriffsabschnitt (209) an der Außenumfangsfläche des elastischen Körpers (21) ausgebildet ist und der Eingriffsabschnitt mit einem an der Seite der Aussparung ausgebildeten Eingriffsabschnitt (207) in Eingriff steht, um ein Herabfallen des elastischen Körpers (21) zu verhindern.

6. Nadelhülseneinheitherstellungsverfahren zur Herstellung der Nadelhülseneinheit (1) nach einem der Ansprüche 1 bis 5, wobei
die Injektionsnadel (31) im Inneren der Aussparung (5) und des Nadeleinführungsdurchgangslochs (7) der Nadelhülse (3) angeordnet wird;
ein Klebstoff (51) in das Nadeleinführungsdurchgangsloch (7) eingefüllt wird; und
der elastische Körper (21) in die Aussparung (5) des Nadelhubs (3) eingeführt wird, wodurch ein Basisabschnitt der Injektionsnadel (31) den elastischen Körper (21) durchdringt.

7. Eine Injektionsvorrichtung, versehen mit:
der Nadelhülseneinheit (1) gemäß einem beliebigen Anspruch aus den Ansprüchen 1 bis 5;
einen Injektionszylinder (41), der in die Aussparung (5) eingeführt ist, bis die Spitze des Injektionszylinders (41) an dem elastischen Körper (21) anliegt; und
einen Kolben (43), der in den Injektionszylinder (41) eingeführt ist, bis die Spitze des Kolbens an dem elastischen Körper (21) anliegt.

## Revendications

1. Unité d'embout d'aiguille (1) pourvue de :
un embout d'aiguille (3) ;
un évidement (5) agencé dans un côté d'extrémité de base de l'embout d'aiguille (3) et dans lequel une partie de pointe d'un cylindre d'injection (41) est insérée ;
un trou traversant d'insertion d'aiguille (7) agencé sur un côté de pointe de l'embout d'aiguille (3) et communiquant avec l'évidement (5) ;
une aiguille d'injection (31) insérée dans l'évidement (5) et le trou traversant d'insertion d'aiguille (7) ; et
un corps élastique (21), qui est agencé intérieurement dans l'évidement (5) depuis un côté d'une ouverture de l'évidement (5) et une surface de pointe du corps élastique (21) vient buter sur une surface inférieure de l'évidement (5), et qui remplit un espace entre la pointe du cylindre d'injection (41) et l'évidement (5),
dans laquelle,
une pointe et une extrémité de base de l'aiguille d'injection (31) sont formées sous une forme d'aiguille ;
des évidements de guidage (21a, 21b) pour guider l'aiguille d'injection (31) sont agencés en position centrale, respectivement, sur chaque surface d'extrémité le long d'une direction vers l'aiguille d'injection (31) du corps élastique (21) ;
un adhésif (51) est rempli à l'intérieur du trou traversant d'insertion d'aiguille (7) ; et
l'extrémité de base de l'aiguille d'injection (31) est introduite dans le corps élastique (21) via l'évidement de guidage (21a), et pénètre à travers le corps élastique (21) via l'évidement de guidage (21b).

2. Unité d'embout d'aiguille (1) selon la revendication 1, dans laquelle,
les évidements de guidage (21a, 21b) sont prévus, respectivement, avec un diamètre sensiblement de la même taille qu'un diamètre extérieur de l'aiguille d'injection (31), ou légèrement supérieur à celui-ci.

3. Unité d'embout d'aiguille (1) selon la revendication 1, dans laquelle,
les évidements de guidage (21a, 21b) sont remplis lorsque le corps élastique (21) est déformé par l'aiguille d'injection (31).

4. Unité d'embout d'aiguille (1) selon la revendication 1, dans laquelle,
l'embout d'aiguille (3) est configuré par un membre unique.

5. Unité d'embout d'aiguille (1) selon une quelconque revendication de la revendication 1 à la revendication 4, dans laquelle,
une partie de mise en prise (209) est agencée sur la surface périphérique externe du corps élastique (21), et la partie de mise en prise est mise en prise avec une partie de mise en prise (207) agencée sur le côté de l'évidement, afin d'empêcher que le corps élastique (21) ne tombe.

6. Procédé de fabrication d'unité d'embout d'aiguille, pour fabriquer l'unité d'embout d'aiguille (1) selon une quelconque revendication de la revendication 1 à la revendication 5, dans lequel,
l'aiguille d'injection (31) a été agencée intérieurement dans l'évidement (5) et le trou traversant d'insertion d'aiguille (7) de l'embout d'aiguille (3) ;
un adhésif (51) est rempli à l'intérieur du trou traversant d'insertion d'aiguille (7) ; et le corps élastique (21) est inséré dans l'évidement (5) de l'embout d'aiguille (3), de sorte qu'une partie de base de l'aiguille d'injection (31) pénètre à travers le corps élastique (21).

7. Dispositif d'injection pourvu de :
l'unité d'embout d'aiguille (1) selon une quelconque revendication de la revendication 1 à la revendication 5 ;
un cylindre d'injection (41) inséré dans l'évidement (5) jusqu'à ce que la pointe du cylindre d'injection (41) vienne buter sur le corps élastique (21) ; et
un piston (43) inséré dans le cylindre d'injection (41) jusqu'à ce que la pointe du piston vienne buter sur le corps élastique (21).
